Europäisches Patentamt

European Patent Office

Office européen des brevets

(19)

(11) Numéro de publication : **0 159 954**
**B1**

(12) **FASCICULE DE BREVET EUROPÉEN**

(45) Date de publication du fascicule du brevet :
**28.09.88**

(51) Int. Cl.⁴ : **C 12 N 13/00, C 12 N 15/00**

(21) Numéro de dépôt : **85430007.6**

(22) Date de dépôt : **15.03.85**

(54) **Procédé perfectionné de fusions de cellules induites par pulsations électriques.**

(30) Priorité : **22.03.84 FR 8404621**

(43) Date de publication de la demande :
**30.10.85 Bulletin 85/44**

(45) Mention de la délivrance du brevet :
**28.09.88 Bulletin 88/39**

(84) Etats contractants désignés :
**AT BE CH DE FR GB IT LI LU NL SE**

(56) Documents cités :
**SCIENCE, vol. 216, no. 4545, 30 avril 1982, pages 537-538, AAAS; J. TEISSIE et al.: "Electric pulse-induced fusion of 3T3 cells in monolayer culture"**
**R.IAN FRESHNEY: "Culture of Animal Cells. A Manual of Basic Technique, 1983, pages 225,237, A.R. Liss, Inc., New York, US;**
**THE JOURNAL OF MEMBRANE BIOLOGY, vol. 67, 1982, pages 165-182, Springer-Verlag, New York, US; U. ZIMMERMANN et al.: "Electric field-induced cell-to-cell fusion"**
**CHEMICAL ABSTRACTS, vol. 100, no. 13, 26 mars 1984, page 313, no. 99361m, Columbus, Ohio, US; C. FINAZ et al.: "Electrofusion - a new, highly efficient technique for generating somtic cell hybrids"**

(73) Titulaire : **Etablissement Public dit: CENTRE NATIO-NAL DE LA RECHERCHE SCIENTIFIQUE (CNRS) 15, Quai Anatole France F-75007 Paris (FR)**

(72) Inventeur : **Teissie, Justin 1 bis rue de Salas F-31520 Ramonville-Sainte-Agne (FR)**

(74) Mandataire : **Rinuy, Santarelli 14, avenue de la Grande Armée F-75017 Paris (FR)**

EP 0 159 954 B1

## Description

La présente invention concerne un procédé perfectionné de fusions de cellules induites par pulsations électriques.

On sait que la technique de fusion cellulaire permet la réalisation de nouvelles espèces hybrides où se retrouvent les caractères des cellules parentales. Cela permet donc de transférer les avantages des deux parents au niveau de l'hybride sous réserve d'une sélection ultérieure. Ainsi la recherche de nouvelles espèces cellulaires alliant les avantages de cellules déjà existantes est un thème prioritaire dans le domaine de la biotechnologie. On peut donc considérer l'hybridation cellulaire comme une forme d'ingénierie génétique.

Au cours des dernières décennies, deux techniques de fusion cellulaire furent développées. Elles font toutes deux appel à des agents exogènes ou fusogènes d'origine virale (comme le virus de Sendaï) ou chimique (comme le polyéthylène glycol : PEG). Les modes d'action de ces procédés restent mal définis et leur caractère empirique n'a conduit qu'à des rendements faibles en hybrides viables (environ un hybride viable pour 100.000 cellules parentales au mieux) du fait que l'agent exogène ou fusogène a souvent un effet létal sur les cellules.

On notera au surplus que l'élimination ultérieure de la substance exogène du milieu de culture reste aléatoire et qu'un autre inconvénient de ces deux techniques réside dans l'absence de la définition de la cible de cette substance exogène.

Plus récemment, on a proposé une nouvelle technique, à savoir celle de l'électrofusion. Les premiers travaux faisaient appel au couplage diélectrophorèse-pulsations électriques appliqué à des cellules en suspension dans un milieu approprié dit « milieu de pulsation » (isotonique pour les cellules ne comprenant aucun ion minéral mais des enzymes protéolytiques).

Dans la plupart des cas, les cellules sont préalablement traitées par des enzymes protéolytiques (de type pronase ou neuramidase) perdant ainsi leurs caractères antigéniques. Des travaux ultérieurs ont montré qu'il était possible d'utiliser la technique de la pulsation électrique directement sur des cellules non préalablement traitées poussant en mono-couches et fixées sur des boîtes de Petri. Dans ces conditions, le milieu de pulsation ne comprend pas d'enzymes protéolytiques mais des ions minéraux notamment du tampon phosphate et du chlorure de magnésium. On a alors constaté qu'un meilleur contact s'établissait naturellement entre les cellules et que l'on obtenait ainsi un taux d'hybridation de l'ordre de 100 fois supérieur comparé à celui obtenu par les techniques antérieures (Exp. Cell Res. vol. 150 (1984) p. 477-482).

Une des théories avancées pour expliquer une telle augmentation de rendement consiste à dire que lorsqu'il est appliqué à une suspension cellulaire, un champ électrique induit un potentiel membranaire qui dépend de la taille des cellules, de leur forme et de l'intensité de ce champ. Or, il est connu que des pores réversibles sont induits par des valeurs intenses des potentiels membranaires, pores créés par la croissance en taille de défauts de structure de la matrice lipidique. On peut donc émettre l'hypothèse que le champ électrique induit un potentiel membranaire dans le cas des cellules en culture et qu'au-delà d'un certain seuil, ce dernier déclenche le phénomène d'ouverture de pores et la création de canaux dont la taille va croître tant que le champ est appliqué. Ces canaux se referment après l'impulsion. Si les cellules sont alors en contact, un mécanisme de fermeture intercellulaire induisant la fusion entre en compétition avec le mécanisme de fermeture intracellulaire classique, ce qui expliquerait la raison pour laquelle la fusion cellulaire est, non seulement possible, mais favorisée. D'où l'importance qu'il faut attribuer à la mise en contact des cellules en culture.

La présente invention se propose donc d'augmenter encore la capacité de cette « électrofusion » en volume de cellules traitées par l'utilisation de cultures sur microporteurs.

Le procédé perfectionné selon l'invention est alors essentiellement caractérisé par le fait qu'il consiste à faire pousser les cellules sur un support sous forme particulaire et à soumettre ledit support ainsi recouvert de* cellules, dans un milieu de pulsation à une série d'impulsions d'un champ électrique d'une façon connue en soi.

Suivant d'autres caractéristiques de l'invention :

— le support est en une substance naturelle ou synthétique, connue pour être non cytotoxique et non inhibitrice de divisions cellulaires ;

— le support est un solide ou un liquide non miscible aux milieux de culture et de pulsation ;

— le support est avantageusement sous forme de billes ou de petites sphères ;

— la dimension particulaire ou la granulométrie du support est avantageusement au-delà de 100 μm ;

— le support est du type résine synthétique choisie parmi les polymères de la classe des polyéthylènes, polydextranes, polystyrènes ;

— le support est choisi parmi les substances minérales, naturelles ou manufacturées du type verre ;

— les milieux de culture et de pulsation sont choisis parmi les milieux généralement utilisés et connus en soi.

D'autres caractéristiques et les avantages de l'invention ressortiront plus clairement de la description qui va suivre donnant un exemple de mise en œuvre de l'invention :

Exemple

Au sein d'une boîte de culture du type connu sous le nom de « boîte de culture Nunc », d'un diamètre de 50 mm, on dispose un lit de billes de polyéthylène d'un diamètre de 200 μm (telles que celles vendues sous le nom de « billes Biosilon »® représentant un volume d'environ 0,2 ml et 1,8 ml d'un milieu de culture à base, par exemple, d'un milieu minimum de Eagle, de 8 % de sérum de veau nouveau-né, pénicilline, streptomycine et L-glutamine. Les cellules dans le cas du présent exemple illustratif de l'invention sont des cellules d'ovaires de hamster chinois (CHO). Il est bien entendu que l'on peut cultiver d'une manière similaire, toutes cellules de même lignée ou de lignées différentes à l'effet d'obtenir la formation aussi bien de polycaryons que d'hétérocaryons.

Après une heure d'incubation à 37° C dans une atmosphère de 5 % $CO_2$ et 95 % air (enceinte Heraeus), on ajoute 1 ml de suspension cellulaire en croissance exponentielle (concentration 3,2 $10^5$ C/ml). La culture est mise à incuber pendant plusieurs jours à 37° C en atmosphère $CO_2$-air. Les billes sont alors récupérées, lavées dans du milieu de pulsation puis remises en suspension dans ce dernier milieu. Celui-ci consiste, par exemple, en 10 mM phosphate, pH 7,2, 1 mM $MgCl_2$ et 250 mM saccharose.

On dépose alors 200 μl de cette suspension de billes dans une cellule de pulsation de tout type approprié comprenant deux électrodes parallèles en acier inoxydable et distantes l'une de l'autre de 5 mm. On soumet la suspension à 5 impulsions d'un champ électrique d'une intensité de 2 kV/cm d'une durée de 100 μ sec. à des intervalles de 1 seconde puis on transfère, par exemple à l'aide de cones « Pipetman » la suspension dans du milieu de culture (environ 2 ml) et on fait incuber pendant 2 heures.

Toutes ces opérations sont conduites à la manière habituelle sous une hotte à flux laminaire.

En vue de leur analyse, on fixe ensuite les cellules, de façon connue en soi, au moyen d'un mélange éthanol/formaldéhyde/acide acétique et on les conserve dans un mélange eau/éthanol.

Pour l'examen, la coloration est réalisée au « bleu de Giemsa » et l'observation, sous microscope inversé, est faite sans contraste de phase et la fusion est visualisée comme on le sait, par l'existence de cellules polynucléées n'existant pas dans la culture initiale.

On constate que l'index de fusion atteint 40 % comparé au niveau spontané de 8 %.

Il va de soi que la présente invention n'a été décrite et illustrée qu'à titre purement explicatif et nullement limitatif et que toute modification utile équivalente pourra y être apportée sans sortir de son cadre.

C'est ainsi que l'exemple ci-dessus n'est absolument limité ni au choix des billes-supports qui n'ont aucun caractère spécifique en elles-mêmes, ni au choix des cellules.

En effet, le procédé de l'invention s'applique à tous les types de support ou systèmes de supports répondant aux critères énoncés ici et sur lesquels les cellules peuvent pousser et établir des contacts cellulaires. Il s'applique aussi à toute culture cellulaire en vue de l'obtention d'hybrides stables et viables et pour l'obtention d'homo- et d'hétérocaryons.

On notera aussi que le procédé de l'invention peut être mis en œuvre sur un milieu dit « en écoulement », comme illustré sur le schéma annexé.

On a représenté sur ce schéma (en 1) un réservoir de stockage du milieu de pulsation dans lequel les billes (B) sont finalement mises en suspension comme décrit à l'exemple ci-dessus. Ce milieu est mis en circulation (écoulement) dans le conduit (2) en régime contrôlé par une mise en dépression contrôlée (3) de l'enceinte de récupération (4) dans laquelle débouche ledit conduit (2). Au cours de cet écoulement, ce milieu est soumis aux impulsions du champ électrique entre les électrodes (E) alimentées par le générateur (G).

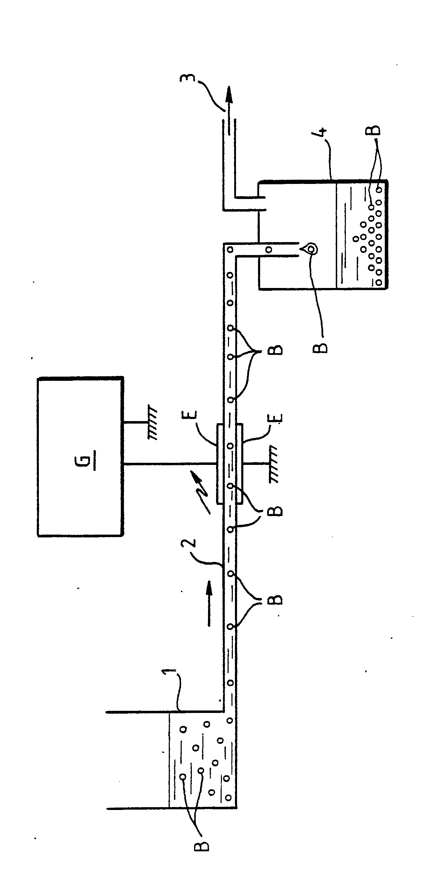

## Revendications

1. Procédé perfectionné de fusions de cellules induites par pulsations électriques, procédé caractérisé par le fait qu'il consiste à faire pousser les cellules sur un support sous forme particulaire et à soumettre ledit support ainsi recouvert de cellules, dans un milieu de pulsation à un série d'impulsions d'un champ électrique d'une façon connue en soi.

2. Procédé selon la revendication 1, caractérisé par le fait que le support est en une substance naturelle ou synthétique connue pour être non cytotoxique et non inhibitrice de divisions cellulaires.

3. Procédé selon la revendication 1, caractérisé par le fait que le support est un solide ou un liquide non miscible aux milieux de culture et de pulsation.

4. Procédé selon l'une des revendications 1 à 3, caractérisé par le fait que le support est sous forme de billes ou de petites sphères.

5. Procédé selon l'une quelconque des revendications 1 à 4, caractérisé par le fait que la dimension particulaire ou la granulométrie du support est avantageusement au-delà de 100 μm.

6. Procédé selon l'une quelconque des revendications 1 à 5, caractérisé par le fait que le support est du type résine synthétique choisie parmi les polymères de la classe des polyéthylènes, polydextranes, polystyrènes.

7. Procédé selon l'une quelconque des revendications 1 à 5, caractérisé par le fait que le support est choisi parmi les substances minérales, naturelles ou manufacturées du type verre.

8. Procédé selon l'une quelconque des revendications 1 à 7, caractérisé par le fait que les milieux de culture et de pulsation sont choisis parmi les milieux généralement utilisés et connus en soi.

## Claims

1. An improved method of cell fusion induced by electric pulsations, characterized in that it consists of growing the cells on a carrier in particulate form, and submitting, in a pulsation medium, the said carrier so covered with cells to a series of impulses of an electric field in a manner known per se.

2. A method according to Claim 1, characterized in that the carrier is a natural or synthetic substance known not to be cytotoxic and not to inhibit cell divisions.

3. A method according to Claim 1, characterized in that the carrier is a solid or a liquid which is not miscible with the culture and pulsation media.

4. A method according to any one of Claims 1 to 3, characterized in that the carrier is in the form of balls or small spheres.

5. A method according to any one of Claims 1 to 4, characterized in that the dimension of the particles or the grain size of the carrier is advantageously above 100 μm.

6. A method according to any one of Claims 1 to 5, characterized in that the carrier is of the synthetic resin kind, chosen from the group of polymers comprising polyethylenes, polydextrans and polystyrenes.

7. A method according to any one of Claims 1 to 5, characterized in that the carrier is chosen from the group comprising inorganic substances, natural or manufactured, which are like glass.

8. A method according to any one of Claims 1 to 7, characterized in that the culture and pulsation media are chosen from the group comprising the media generally employed and known per se.

**Patentansprüche**

1. Verbessertes Verfahren zur durch elektrische Pulse induzierten Zellverschmelzung, dadurch gekennzeichnet, daß die Zellen auf einem Träger besonderer Form wachsen gelassen werden und dieser mit Zellen bedeckte Träger dann in einem Pulsationsmilieu in an sich bekannter Weise einer Serie von Impulsen eines elektrischen Feldes unterworfen wird.

2. Verfahren nach Anspruch 1, dadurch gekennzeichnet, daß der Träger aus einer natürlichen oder künstlichen Substanz besteht, die bekannterweise nicht zytotoxisch ist und die Zellteilung nicht behindert.

3. Verfahren nach Anspruch 1, dadurch gekennzeichnet, daß der Träger ein Festkörper oder eine Flüssigkeit ist, die mit dem Kulturmilieu oder Pulsationsmilieu nicht mischbar ist.

4. Verfahren nach einem der Ansprüche 1 bis 3, dadurch gekennzeichnet, daß der Träger in Form von Granülen oder Kügelchen vorliegt.

5. Verfahren nach einem der Ansprüche 1 bis 4, dadurch gekennzeichnet, daß die besondere Dimension oder Granulometrie des Trägers vorteilhaft über 100 μm liegt.

6. Verfahren nach einem der Ansprüche 1 bis 5, dadurch gekennzeichnet, daß der Träger ein synthetisches Harz, ausgewählt aus der Gruppe der Polyäthylene, Polydextrane und Polystyrole, ist.

7. Verfahren nach einem der Ansprüche 1 bis 5, dadurch gekennzeichnet, daß der Träger aus den natürlichen oder künstlichen, glasartigen Mineralsubstanzen ausgewählt ist.

8. Verfahren nach einem der Ansprüche 1 bis 7, dadurch gekennzeichnet, daß das Kultur- und Pulsationsmilieu unter den im allgemeinen verwendeten und an sich bekannten Milieus ausgewählt ist.